# EUROPÄISCHE PATENTSCHRIFT

(11) **EP 3 412 681 B1**
(45) Veröffentlichungstag und Bekanntmachung des Hinweises auf die Patenterteilung: **17.04.2024**
(21) Anmeldenummer: 18175770.9
(22) Anmeldetag: 30.04.2014
(51) Int. Cl.: C07K 14/005, C12N 15/867, A61K 38/16

(54) **PROTRANSDUZIN B EIN ENHANCER DES GENTRANSFERS**
PROTRANSDUCIN B AN ENHANCER OF GENE TRANSFER
PROTRANSDUZIN-B EN TANT QU'UN ACTIVATEUR DE TRANSCRIPTION DU TRANSFERT DE GÈNES

(30) Priorität: 02.05.2013 EP 13166266
(43) Veröffentlichungstag der Anmeldung: 12.12.2018
(62) Teilanmeldung aus: 14724030.3
(73) Patentinhaber: Pharis Biotec GmbH, 30625 Hannover (DE)
(72) Erfinder: Forssmann, Wolf-Georg, 30625 Hannover (DE); Zgraja, Andreas, 30519 Hannover (DE); Richter, Rudolf, 61462 Königstein (DE)
(74) Vertreter: dompatent von Kreisler Selting Werner - Partnerschaft von Patent- und Rechtsanwälten mbB

(56) Entgegenhaltungen:
- EP-A1- 2 452 947
- EP-A2- 2 334 318
- WO-A1-2011/034207
- MARAL YOLAMANOVA ET AL: "Peptide nanofibrils boost retroviral gene transfer and provide a rapid means for concentrating viruses", NATURE NANOTECHNOLOGY, Bd. 8, Nr. 2, 1. Februar 2013 (2013-02-01) , Seiten 130-136, XP055086479, ISSN: 1748-3387, DOI: 10.1038/nnano.2012.248
- DAMEN M ET AL: "Delivery of DNA and siRNA by novel gemini-like amphiphilic peptides", JOURNAL OF CONTROLLED RELEASE, ELSEVIER, AMSTERDAM, NL, Bd. 145, Nr. 1, 1. Juli 2010 (2010-07-01), Seiten 33-39, XP027065026, ISSN: 0168-3659, DOI: 10.1016/J.JCONREL.2010.03.028 [gefunden am 2010-04-08]
- S. JAWHAR ET AL: "Pyroglutamate Amyloid- (A ): A Hatchet Man in Alzheimer Disease", JOURNAL OF BIOLOGICAL CHEMISTRY, Bd. 286, Nr. 45, 11. November 2011 (2011-11-11), Seiten 38825-38832, XP055086822, ISSN: 0021-9258, DOI: 10.1074/jbc.R111.288308

## Beschreibung

Gegenstand der vorliegenden Anmeldung ist ein N-terminal geschütztes Peptid, ein Arzneimittel enthaltend das Peptid, das Peptid zur Verwendung in der Gentherapie, ein Verfahren zur Verstärkung der Infektion einer Zelle mit einem gentechnischen Viruskonstrukt, eine Verwendung des Peptids zur Amplifizierung zur Transfection bzw. Transduction.

Die Bedeutung der Gentechnologie hat in den letzten Jahren durch enorme Fortschritte in den angewandten Methoden zugenommen, da nicht nur die Herstellung von Protein-Peptid-Wirkstoffen sondern auch die Transfection von Zellen mit stabilen Genen als Labortool und zuletzt der Einschleusung von Genen in Zellen als Ersatz für Gendefekte von grosser Tragweite für die Therapie von zahlreichen Erkrankungen absehbar ist.

Die Einschleusung von Genmaterial zur Veränderung spezifischer Zellfunktionen ist seit der Klonierung der ersten humanen Gene und der rekombinanten Produktion ein unverzichtbares Tool der biologisch-medizinischen Grundlagen- und Anwendungsforschung geworden, das die Methoden der Effizienz des Gentransfers einen ständigen Fortschritt durchmachen. Zahlreiche Methoden der Geneinschleusung haben zur Optimierung geführt. Erfahrungen, die über eine langjährige, zunächst sehr langsame Geschichte gesammelt wurden.

Schon vor der Aufklärung der Funktion von Desoxyribonukleinsäure (DNS) 1953 durch F. Crick und J. Watson, war es Ende der 1920'er Jahre F. Griffith in Experimenten gelungen, apathogene Pneumokokken-Stämme in Pathogene zu transformieren. Diese Transformation war einem glücklichen Umstand zu verdanken, da die Pneumokokken über eine seltene natürliche Kompetenz der DNS Aufnahme verfügten. Ein gezieltes Einschleusen der DNS in Prokaryonten gelang u.a. J. Lederberg, M. Delbrück und S. Luria mithilfe von Phagen, die so genannte Transduction. Mit der Etablierung der Zellkultur, dem Kultivieren von Eukaryonten Zellen unter *in vitro* Bedingungen, wurden eine Reihe von physikalischen und chemischen Verfahren zur Transfection entwickelt. Zu den häufiger genutzten, aber apparativ aufwendigeren, physikalischen Methoden gehören die Elektroporation und die Mikroinjektion, die mit den von der Anwendung einfacheren chemischen Verfahren konkurierten, wie die in den 80'er Jahren und auch noch heute übliche Calcium-Phosphat-Präzipitation Methode oder die in den frühen 90'er Jahren weit verbreiteten Methoden, die auf kationische Lipide bzw. kationische Polymere basierten. Der Einsatz dieser Verfahren war aber immer von den Zellen bzw. der DNS abhängig. Auch lag die in die Zellen eingeschleuste DNS im Allgemeinen extrachromosomal vor (transiente Transfection) und wurde so nicht an die Tochterzellen weiter gegeben. Von Phagen (z.B. Lambda Phage) wusste man aber, dass diese auch ihre DNS ins Wirtsgenom integrieren können (Prophage, lysogener Infektionsweg). Von hieraus war es nur noch ein kleiner Schritt zur (1981/1982) "Etablierung von Retroviren als Genvektoren" (durch Doehmer et al. und Tabin et al.). Viren sind Spezies- und Organ-/Gewebe- spezifisch, weshalb nicht alle Viren alle (Eukaryonten) Zellen infizieren. Veränderungen der Virus Hülle (Austausch von Glykoproteinen, sogenannte Pseudotyped Viren) sowie Zusätze von meist kationischen Peptiden soll die Transductions Effizienz steigern.

Erste Verstärker der Aufnahme von Viruspartikeln sind bei der Untersuchung von HIV aufgefallen. Bei Analysen der *in vitro* Infektion mittels eines spezifischen Zelltests wurde die Hemmung der Fusion von HIV durch Blutfiltratpeptide beobachtet. (Münch et al, VIRIP).

Es wurde gezeigt, dass diese überraschenderweise natürlich vorkommenden Fragmente von Proteinen im menschlichen Sperma faserartige Strukturen als enhancer - *"Semen derived Enhancer of Virus Infection"* (SEVI) ausbilden, die als Amyloidfibrillen gekennzeichnet sind. Diese Nanofibrillen verstärken das Andocken von Viren an ihre Zielzellen und erhöhen die Rate der Virusinfektion um mehrere log-Stufen.

Dies wurde genutzt, um einen retroviralen Gentransfer für die Grundlagenforschung und mögliche zukünftige therapeutische Anwendungen zu verbessern. So konnte gezeigt werden, dass lentivirale und gamma-retrovirale Vektoren, die für die Gentherapie verwendet werden, in der Anwesenheit des SEVI - Proteins eine vielfach höhere Gen-Transferrate bei unterschiedlichen Zelltypen aufweisen, wie z.B. humanen T-Zellen, Zervixkarzinomzellen, Leukämiezellen, hämatopoietischen Stammzellen und embryonalen Stammzellen (Wurm et al., J Gene Med. 2010, 12, 137-46; Wurm et al., Biol Chem. 2011, 392, 887-95).

Untersuchungen zur Entwicklung weiterer Enhancer wie z.B. dem SEVI und Seminogelin führten zu der Annahme, dass Peptide aus viralen Hüllproteinen auch als Enhancer der Transfection geeignet sein könnten, was überraschenderweise von unerwartet gutem Erfolg war (Maral Yolamanova, Nature Nanotechnology). So konnte z.B. gezeigt werden, dass HI-Viren, welche mit verschiedenen Konzentrationen (1-100 µg/ml) Protransduzin A (Synonym: EF-C) vorinkubiert wurden, eine um mehrere log-Stufen stärkere Infektionsrate bei Reporterzellen aufweisen. Als Wirkmechanismus wurde angenommen, dass EF-C fibrilläre Strukturen ausbildet, die in der Lage sind, Viren zu binden, zu konzentrieren und entprechend den Zelleintritt der Viren zu vervielfachen. EF-C verstärkt neben der Infektion von Viruspartikeln mit hoher Effizienz die Transduction von lentiviralen und retroviralen Partikeln in verschiedensten humanen Zelltypen (T-Zellen, Gliazellen, Fibroblasten, hämatopoietische Stammzellen), welche in der Gentherapie Anwendung finden (Jan Münch et al., Nature Nanotechnology, Bd. 8, Nr. 2, S. 130-136). Protransduzin A ist auch Gegenstand der EP 2 452 947 A1.

Aufgrund der oben dargestellten zunehmenden Bedeutung der Gentechnologie sind effektivere Verstärker des Gentransfers wünschenswert. Das der Erfindung zu Grunde liegende Problem ist die Bereitstellung eines verbesserten Verstärkers des Gentransfers.

Überraschenderweise wurde gefunden, dass ein N-terminal geschütztes Peptid mit der Sequenz
X-Glu-Cys-Lys-Ile-Lys-Gln-Ile-Ile-Asn-Met-Trp-Gln (SEQ ID NO 1),
wobei X eine den N-Terminus des Peptids schützende Gruppe ist, das der Erfindung zu Grunde liegende Problem löst. Erfindungsgemäß stellt X eine oder zwei Alkylgruppen, wie Methyl-, Ethyl-, Propyl- oder Butylgruppen, eine Acylgruppe, wie eine Acetyl- oder Propionylgruppe dar.

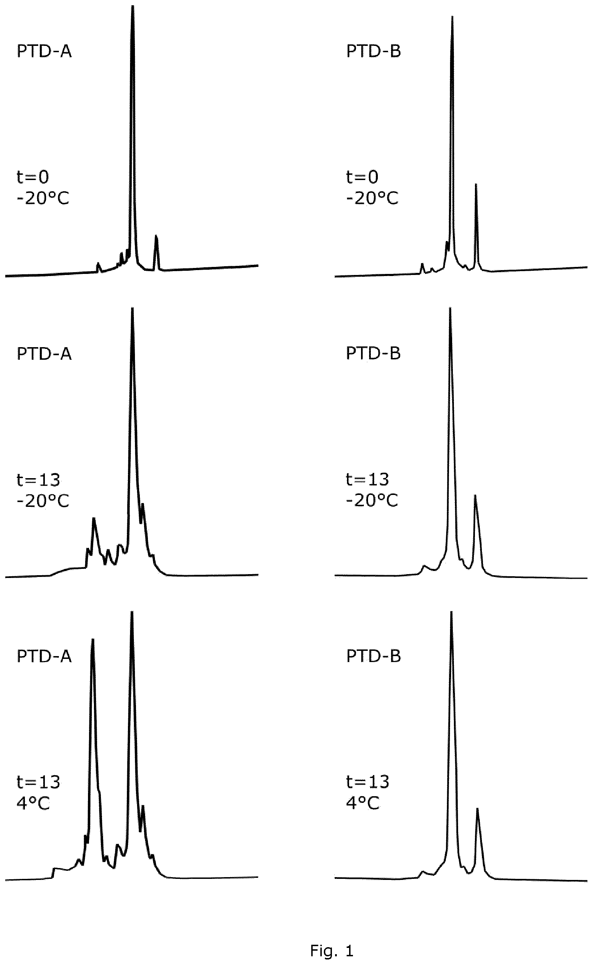

Überraschenderweise hat sich gezeigt, dass die Modifikation des N-terminalen Endes in vitro (ohne zelluläre Einflüsse, insbesondere Gegenwart von Enzymen) zu einer enormen Stabilitätserhöhung des Protransduzin in wässriger Lösung führt. Dies wird aus den Ergebnissen, die in Figur 1 wiedergegeben sind, deutlich.

In der linken Spalte der Figur 1 (HPLC-Chromatogramm) werden Ergebnisse für Protransduzin A bei einer Lagerung von 0-13 Tagen, bei -20 °C und bei 4 °C (13 Tagen) den Ergebnissen für Protransduzin B unter entsprechenden Bedingungen gegenübergestellt. Es wird deutlich, dass Protransduzin A bei einer Lagerung von 13 Tagen bei 4 °C fast zur Hälfte abgebaut wird, wohingegen Protransduzin B bei entsprechenden Lagerungsbedingungen nur unwesentlich abgebaut wird (die Höhe der Peaks entspricht der Konzentration der in der Probe enthaltenen Komponenten).

S. Jawhar et al. berichtet in Journal of Biological Chemistry, Bd. 286, Nr. 45, S. 38825-38832 über den Stand der Wissenschaft hinsichtlich Amyloid-Peptiden, insbesondere Pyroglutamat modifizierter Amyloid-Polypeptide. Diese Amyloid Polypeptide weisen eine Vielzahl von Aminosäuren auf und sind mit kurzkettigen Peptiden, zu denen auch die erfindungsgemäßen gehören, grundsätzlich nicht vergleichbar. Im übrigen bezieht sich der Mini Review auf zelluläre Geschehnisse, die sich unter *in vivo* Bedingungen in Gegenwart von Enzymen ereignen, was aber mit den Bedingungen, unter denen die Stabilität von Protransduzin erfindungsgemäß verbessert wurde, also *in vitro* Bedingungen, in keiner Weise verglichen werden kann.

Das erfindungsgemäße Peptid kann auch als Arzneimittel verwendet werden.

Gegenstand der Erfindung ist auch die Verwendung des erfindungsgemäßen Peptids in der Gentherapie zur Behandlung von mit Gentherapie behandelbaren Erkrankungen.

Ebenfalls Gegenstand der Erfindung ist ein Verfahren zur Verstärkung der Infektion einer Zelle mit einem Virus mit den Schritten:
- Bereitstellen des Peptids gemäß Anspruch 1 gelöst in einem organischen Lösungsmittel,
- Zugabe des Peptids zu einer wässrigen Lösung, unter Bildung von unlösbaren Aggregaten des Peptids,
- Vermischen der Lösung aus dem vorhergehenden Schritt und
- Kultivieren der Zellen.

Auch die Verwendung des erfindungsgemäßen Peptids zur Verstärkung der Infektion einer Zelle mit einem Virus Gegenstand der vorliegenden Erfindung.

Schließlich wird auch eine Zusammenstellung enthaltend das erfindungsgemäße Peptid beansprucht.

Das Peptid (Protransduzin B) kann beispielsweise mit der Methode nach Merrifield mit Fmoc geschützten Aminosäuren durchgeführt werden.

Dabei wird mit Fmoc-geschützten Derivaten gearbeitet, also mit (9-fluorenylmethoxycarbonyl) -geschützten Aminosäuren in einer schrittweisen Festphasensynthese nach dem Merrifield-Prinzip, insbesondere auf einem mit Fmoc-L-Glutamin vorbeladenem Wang-Harz (0,59 mmol/g, 100 - 200 mesh) als festem Träger am Synthesizer ABI-433.

Die Aktivierung der Fmoc-L-Aminosäuren, die typischerweise in 10-fachem molaren Überschuss eingesetzt wurden, werden mit [(2-(1H-Benzotriazol-1-yl)-1,1,3,3-tetramethyluronium-hexafluorophosphat] (HBTU, 100 mmol/l) unter Zusatz von 0.5 M 1-Hydroxy-benzotriazol (HOBt) und 2M Diisopropylethylamin (DIEA) in N-Methyl-2-pyrrolidinon (NMP) bei Raumtemperatur durchgeführt.

Die Acylierungsreaktionen dauern im einzelnen 45 Minuten, die Abspaltung der Fmoc- Schutzgruppe mit 20 %igem Piperidin dauert 15 Minuten.

Folgende Aminosäurederivate sowie dazugehörige orthogonal-säurelabile Seitenkettenschutzgruppen werden zur Synthese eingesetzt:
Fmoc-L-Asn(Trt), Fmoc-L-Cys(Trt), Fmoc-L-Gln(Trt), Fmoc-L-Ile, Fmoc-L-Lys(Boc), Fmoc-L-Met und Fmoc-L-Trp(Boc).

Nach Abspaltung des Harzträgers vom Peptidylharz mit 94 %iger Trifluoressigsäure (TFA); 3%igem Ethandithiol (EDT) und 3 %igem entmineralisiertem Wasser wird das Rohpeptid in kaltem tert. Butylmethylether ausgefällt, das Rohpeptid als Sediment abzentrifugiert, der Überstand verworfen.

Die anschließende chromatographische Aufreinigung des Rohpeptides erfolgt präperativ per Gradientenelution.

### Reinigung:

Präparative Trennung: Die Reinigung wird auf einer HPLC der Firma Gilson durchgeführt. Der UV / VIS Detector ist von der Firma Kronwald, die Trennung wird mit der Wellenlänge 230 nm detektiert. Die Flussrate ist 40 ml/min.

Die Säule ist eine Waters Prep-Pak C18-Kartusche (47 x 300 mm).

Eluent A: 0.1 % TFA in entmineralisiertem Wasser; Eluent B: 0.1 % TFA in 80 % Acetonitril und 20% entmineralisiertem Wasser.

Der Gradient für Protransduzin B ist 35 %-55% B in 40 min. das sind 0.5 % B pro Minute.

Das Protransduzin B eluiert bei 40 % B und wird in mehreren Fraktionen à 0.5 bis 1 min. gesammelt. Die nach Analytik sauberen Fraktionen werden gepoolt und lyophilisiert.

### Aufbereitung zur Anwendung:

### Lyophilisation:

Zur Gefriertrocknung wird die Anlage Epsilon 1/45 der Firma Christ benutzt, deren technische Daten wie folgt eingestellt sind: Stellfläche 3,78 m² ; Eisaufnahmevermögen ca. 60 kg; Eiskondensatorleistung max. 45 kg/24 h; Endpartialdruck der Vakuumpumpe 1 × 10⁻³ / 10⁻⁴ mbar mit / ohne Gasballast Gefriertrocknungsdaten (Anlage manuell mit Gasballast betrieben): Endpartialdruck 1 × 10⁻² mbar; Eiskondensatortemperatur- 50° C; Stellflächentemperatur + 15° C; Schaltpunkt der Stellflächenheizung 0,5 mbar; Gefriertrocknungszeit bis 3 Tage.

### Transduction von Zellen mit Protransduzin-B

0,5 mg Protransduzin-B in 50 µl DMSO lösen. Danach zu der Lsg. 450 µl PBS zufügen, dabei entstehen innerhalb von 3 min. Fibrillen. Diese Stammlösung (1 mg/ml) zu den Vektoren zugegeben, so dass man eine Konzentration an Protransduzin-B von 25µg/ml erhält. Die Lösung für 1 min. vortexen und dann bei 5000g 5 min. abzentrifugieren. Der Überstand wird verworfen und das Pellet in etwas PBS aufgeschwemmt und zu den Zellen gegeben. Die Zellen inkubiert man 2 Tage im Brutschrank.

Die Transductionsrate wird durch Protransduzin-B deutlich gesteigert. Bis zu 96 % der Zellen können mit Hilfe von Protransduzin-B transduziert werden.

## Patentansprüche

1. Peptid mit der Sequenz
X-Glu-Cys-Lys-Ile-Lys-Gln-Ile-Ile-Asn-Met-Trp-Gln,
wobei X eine oder zwei Alkylgruppen, wie Methyl-, Ethyl-, Propyl- oder Butylgruppen, oder eine Acylgruppe, wie eine Acetyl- oder Propionylgruppe ist.

2. Arzneimittel enthaltend ein Peptid gemäß Anspruch 1.

3. Peptid nach Anspruch 1 zur Verwendung in der Gentherapie zur Behandlung von mit Gentherapie behandelbarer Erkrankungen.

4. Verfahren zur Verstärkung der Infektion einer Zelle mit einem Virus mit den Schritten:
- Bereitstellen des Peptids gemäß Anspruch 1 gelöst in einem organischen Lösungsmittel,
- Zugabe des Peptids zu einer wässrigen Lösung, unter Bildung von unlösbaren Aggregaten des Peptids,
- Vermischen der Lösung aus dem vorhergehenden Schritt und
- Kultivieren der Zellen.

5. In-vitro Verwendung des Peptids gemäß Anspruch 1 zur Verstärkung der Infektion einer Zelle mit einem Virus.

6. Zusammenstellung enthaltend ein Peptid gemäß Anspruch 1.

## Claims

1. A peptide having the sequence
X-Glu-Cys-Lys-Ile-Lys-Gln-Ile-Ile-Asn-Met-Trp-Gln,
wherein X is one or two alkyl groups, such as methyl, ethyl, propyl or butyl groups, or an acyl group, such as an acetyl or propionyl group.

2. A medicament containing a peptide according to claim 1.

3. The peptide according to claim 1 for use in gene therapy for treating diseases that are treatable with gene therapy.

4. A method for enhancing the infection of a cell by a virus, comprising the steps:
- providing the peptide according to claim 1 dissolved in an organic solvent;
- adding the peptide to an aqueous solution to form insoluble aggregates of the peptide;
- mixing the solution from the preceding step; and
- culturing the cells.

5. In vitro use of the peptide according to claim 1 for enhancing the infection of a cell with a virus.

6. A kit containing a peptide according to claim 1.

## Revendications

1. Peptide avec la séquence
X-Glu-Cys-Lys-Ile-Lys-Gln-Ile-Ile-Asn-Met-Trp-Gln,
où X représente un ou deux groupes alkyle, tels que des groupes méthyle, éthyle, propyle ou butyle, ou un groupe acyle, tel qu'un groupe acétyle ou propionyle.

2. Médicament contenant un peptide selon la revendication 1.

3. Peptide selon la revendication 1 à utiliser dans la thérapie génique pour traiter des maladies traitable par la thérapie génique.

4. Procédé pour renforcer l'infection d'une cellule avec un virus, comprenant les étapes consistant à :
- procurer le peptide selon la revendication 1 dissout dans un solvant organique,
- ajouter le peptide à une solution aqueuse pour former des agrégats insolubles dudit peptide,
- mélanger la solution provenant de l'étape précédent, et
- cultiver les cellules.

5. Utilisation in vitro du peptide selon la revendication 1 pour renforcer l'infection d'une cellule avec un virus.

6. Trousse contenant un peptide selon la revendication 1.
